# EUROPEAN PATENT APPLICATION

(11) **EP 2 589 595 A1**
(43) Date of publication of application: **08.05.2013**
(21) Application number: 11795738.1
(22) Date of filing: 14.06.2011
(51) Int. Cl.: C07D 233/61, A61K 31/4164, A61K 31/4178, A61K 31/454, A61K 31/5383, A61K 31/7056, A61K 38/21, A61P 31/14, A61P 43/00, C07D 233/66, C07D 401/10, C07D 401/14, C07D 403/10, C07D 405/12, C07D 409/14, C07D 471/04, C07D 491/10, C07D 498/04

(54) **AGENT FOR TREATING HCV INFECTION**

(30) Priority: 15.06.2010 JP 2010135742
(71) Applicant: Eisai R&D Management Co., Ltd., Tokyo 112-8088 (JP)
(72) Inventor: YANAGIMACHI Mamoru, Tsukuba-shi Ibaraki 300-2635 (JP); INO Mitsuhiro, Tsukuba-shi Ibaraki 300-2635 (JP)
(74) Representative: HOFFMANN EITLE
(86) International application number: PCT/JP2011/063602
(87) International publication number: WO 2011/158833

(57) **Abstract**

Provided is an imidazolylbenzene compound or salt thereof that controls HCV replication and in addition is particularly capable of strongly controlling HCV replication, and is very effective in the prevention and treatment of HCV infection when used in combination with another agent for treating HCV infection such as Interferon.

## Description

### Technical Field

The present invention relates to a therapeutic agent for HCV infectious disease. More specifically, it relates to a therapeutic agent for HCV infectious disease comprising a compound having an imidazolylbenzene structure.

### Background Art

Hepatitis C virus (hereunder, "HCV"), discovered in 1989 as the major causative virus of non-A, non-B hepatitis following blood transfusion, is an enveloped, single-stranded RNA virus, its genome consisting of single-stranded (+)RNA, and it is classified in the genus *Hepacivirus* of the Flavivirus family. It is estimated that 100 million to 200 million people worldwide are infected with HCV. However, because HCV is able to evade the immune systems of host, HCV-positive persons usually become chronic hepatitis status, then, progressing to hepatic cirrhosis or hepatic cancer. Approximately 90% of hepatic cancer cases are associated with HCV infection, and the death of a large number of patients by hepatic cancer each year is attributed to HCV infection.

Known therapeutic methods for HCV infectious disease include plasma apheresis therapy in which patient blood is returned to the body after removal of HCV, liver supporting therapy by improving the liver function with the treatment of glycyrrhizin or ursodeoxycholic acid, to prevent aggravation of hepatitis, and antiviral therapy in which an antiviral agent such as Interferon or Peginterferon is administered to eliminate HCV from the body with the target of complete cure.
It is known that the most effective therapeutic method for HCV infectious disease is a combination of Peginterferon and ribavirin (1-β-D-riboniranosyl-1H-1,2,4-triazole-3-carboxamide) (Non-patent document 1 and Non-patent document 2). It has showed that sphingolipid biosynthesis and cholesterol biosynthesis are involved in HCV infection, and therefore the use of substances that inhibit the activity or expression of enzymes involved in sphingolipid biosynthesis or cholesterol biosynthesis has been proposed to use the prevention or therapy against the HCV infectious disease (Patent document 1 and Patent document 2).

### Citation List

### Patent Literature

[Patent document 1]
   International Patent Publication No. WO2006/0166657
[Patent document 2]
   International Patent Publication No. WO2007/099869

### Non Patent Literature

[Non-patent document 1]
   Glue. P. et al., Hepatology, 32, 647-653(2000)
[Non-patent document 2]
   Reddy, K.R. et al., Hepatology, 33, 33-4338(2001)

### Summary of Invention

### Technical Problem

Most of the existing therapeutic methods for HCV infectious disease, however, are symptomatic treatment so that it seems to be difficult to reach a complete cure for HCV infectious disease. Interferon, which is the most effective therapeutic method, has a very low effect against HCV 1b genotype, and it has therefore not been promising as a satisfactory therapeutic effect. Combination therapy with Peginterferon and ribavirin is associated with problematic side-effects such as general malaise, anorexia, influenza-like symptoms, and thus the current therapeutic methods cannot be considered adequate. A strong demand therefore exists for establishing an effective therapeutic method for HCV infectious disease which has an excellent therapeutic effect and reduced side-effects. The objective of the present invention is to provide a novel therapeutic agent for HCV infectious disease.

### Solution to Problem

As a result of diligent research with the aim of providing a novel therapeutic agent for HCV infectious disease, the present inventors have completed this invention based on the finding that, surprisingly, an imidazolylbenzene compound or its salt (International Patent Publication No. WO2005/115990 and International Patent Publication No. WO2007/060821) inhibit HCV replication, and further that combination of other therapeutic agents for HCV infectious disease, such as Interferon, with a therapeutic agent for HCV infectious disease comprising an imidazolylbenzene compound or its salt, exhibits particularly potent inhibition on HCV replication.

Specifically, the invention provides the following:
[1] A therapeutic agent for HCV infectious disease comprising a compound represented by formula (I): 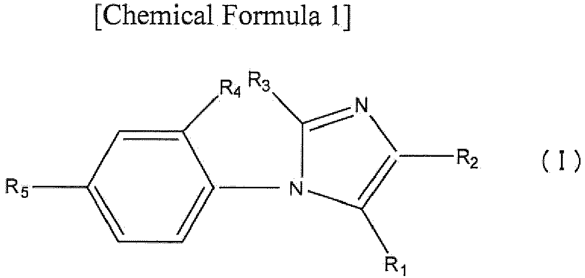 [wherein R₁ represents a hydrogen atom, a halogen atom or C₁₋₆ alkyl,
   R₂ represents a hydrogen atom, C₁₋₆ alkyl or C₁₋₆ alkoxy-C₁₋₄ alkyl,
   R₃ represents a hydrogen atom, C₁₋₆ alkyl or C₁₋₆ alkoxy-C₁₋₄ alkyl,
   R₄ represents a hydrogen atom, a halogen atom, C₁₋₆ alkyl, C₁₋₆ alkoxy, C₃₋₆ cycloalkoxy, C₁₋₆ alkylsulfonyl, C₁₋₆ alkoxy-C₁₋₄ alkyl, hydroxy(C₁₋₆)alkyl, halo(C₁₋₆)alkyl, amino, formyl, C₂₋₄ alkanoyl, nitro or cyano, and
   R₅ represents a group represented by the formula: 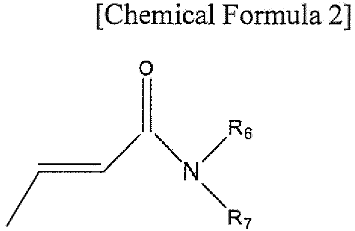 (wherein R₆ represents indanyl, chromanyl, picolyl, C₇₋₁₅ aralkyl, C₃₋₆ cycloalkyl-C₁₋₄ alkyl or N-C₇₋₁₅ aralkylamino, optionally having a substituent selected from the group consisting of halogen atom(s), C₁₋₆ alkyl, halo(C₁₋₆)alkyl, C₁₋₆ alkoxy, C₃₋₆ cycloalkylene, pyrazolyl and C₆₋₁₄ aryl,
   and R₇ represents a hydrogen atom, C₁₋₆ alkyl or hydroxy(C₁₋₆)alkyl, or R₆ and R₇, together with the nitrogen atom to which they are bonded, represent a group shown in the following table:

**[Table 1]**

| # | - N (R6)(R7) |
|---|---|
| 1 | 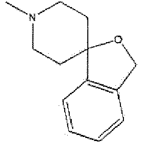 |
| 2 | 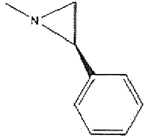 |
| 3 | 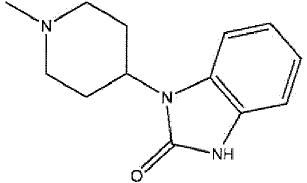 |

or a group shown in the following table:

**[Table 2]**

| # | R₅ | # | R₅ |
|---|---|---|---|
| 1 | 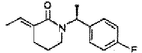 | 7 | 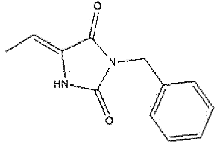 |
| 2 | 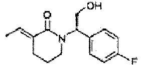 | 8 | 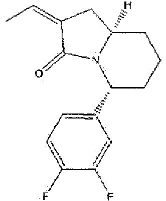 |
| 3 | 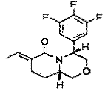 | 9 | 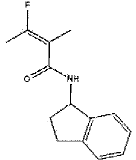 |
| 4 | 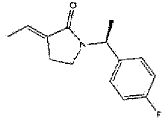 | 10 | 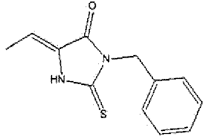 |
| 5 | 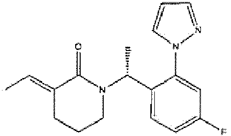 | 11 | 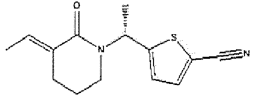 |
| 6 | 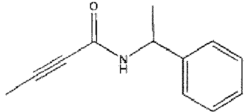 | 12 | 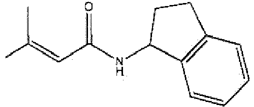 |

)] or a pharmaceutically acceptable salt thereof;
[2] The therapeutic agent for HCV infectious disease comprising the compound represented by formula (I): 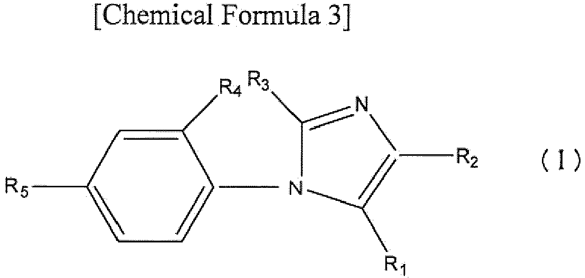 [wherein R₁, R₂, R₃, R₄ and R₅ have the same meanings as in claim 1] or the pharmaceutically acceptable salt thereof according to [1] above, which is for use in combination with another therapeutic agent for HCV infectious disease;
[3] The therapeutic agent for HCV infectious disease comprising the compound represented by formula (I): 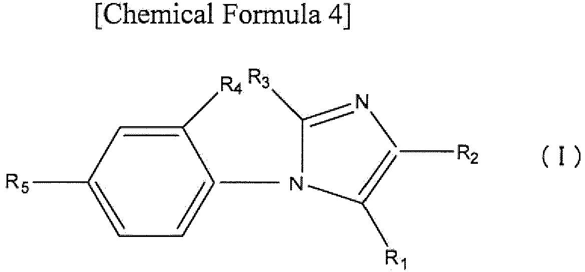 [wherein R₁, R₂, R₃, R₄ and R₅ have the same meanings as in claim 1]
   or the pharmaceutically acceptable salt thereof according to [1] or [2] above, which is for administration either simultaneously or successively with another therapeutic agent for HCV infectious disease;
[4] The therapeutic agent for HCV infectious disease comprising the compound represented by formula (I) or the pharmaceutically acceptable salt thereof, and another therapeutic agent for HCV infectious disease;
[5] The therapeutic agent for HCV infectious disease according to any one of [1] to [5] above, wherein the compound represented by formula (I) or the pharmaceutically acceptable salt thereof is a compound selected from the group consisting of:
   (E)-1-[(1S)-1-(4-fluorophenyl)ethyl]-3-[3-methoxy-4-(4-methyl-1H-imi dazol-1-yl)benzylidene]piperidin-2-one,
   (E)-1-[(1R)-1-(4-fluorophenyl)-2-hydroxyethyl]-3-[3-methoxy-4-(4-met hyl-1H-imidazol-l-yl)benzylidene]piperidin-2-one,
   (E)-(4R,9aS)-7-[3-methoxy-4-(4-methyl-1H-imidazol-1-yl)benzylidene] -4-(3,4,5-trifluorophenyl)hexahydropyrido[2,11-c][1,4]oxazin-6-one, 3-[3-methoxy-4-(4-methyl-1H-imidazol-1-yl)benzylidene]-1-[(1S) -1-phenylethyl]piperidin-2-one,
   (E)-N-cyclohexylmethyl-3-[4-(1H-imidazol-1-yl)-3-methoxyphenyl]acr ylamide,
   (E)-3-[4-(1H-imidazol-1-yl)-3-methoxyphenyl]-N-(1-phenylcyclopropy l)acrylamide,
   (E)-3-[4-(1H-imidazol-1-yl)-3-trifluorophenyl]-N-indan-1-yl-acrylamid e,
   (E)-3-[3-acetyl-4-(1H-imidazol-1-yl)-phenyl]-N-indan-1-yl-acrylamide,
   (E)-3-[3-fluoro-4-(1H-imidazol-1-yl)-phenyl]-2-butenoic acid indan-1-yl-amide,
   (E)-3-[4-(1H-imidazol-1-yl)-3-methoxyphenyl]-N-(1,2,3,4-tetrahydrona phthalen- 1-yl)acrylamide, (E)-3-[4-(1H-imidazol-l-yl)-3-methoxymethylphenyl]-N-indan-l-yl-acr ylamide,
   and their pharmaceutically acceptable salts;
[6] The therapeutic agent for HCV infectious disease according to any one of [2] to [5] above, wherein the other therapeutic agent for HCV infectious disease is Interferon;
[7] The therapeutic agent for HCV infectious disease according to [6] above, wherein the Interferon is Interferon-α-2b;
[8] The therapeutic agent for HCV infectious disease according to any one of [2] to [5] above, wherein the other therapeutic agent for HCV infectious disease is Ribavirin;
[9] The therapeutic agent for HCV infectious disease according to [2] or
[8] above, wherein the other therapeutic agent for HCV infectious disease is both Interferon and Ribavirin;
[10] The therapeutic agent for HCV infectious disease according to any one of [2] to [5] above, wherein the other therapeutic agent for HCV infectious disease is an HCV protease inhibitor or HCV polymerase inhibitor;
[11] A kit for therapy of HCV infectious disease, which comprises the compound represented by formula (I) or the pharmaceutically acceptable salt thereof according to [1] above, and another therapeutic agent for HCV infectious disease;
[12] A use of the compound represented by formula (I) or the pharmaceutically acceptable salt thereof according to [1] above, for production of a therapeutic agent for HCV infectious disease comprising the compound or the pharmaceutically acceptable salt thereof;
[13] The use according to [12] above, wherein the therapeutic agent for HCV infectious disease comprises the compound or the pharmaceutically acceptable salt thereof, and another therapeutic agent for HCV infectious disease.
[14] A use of the compound represented by formula (I) or the pharmaceutically acceptable salt thereof according to [1] above for production of a therapeutic agent for HCV infectious disease comprising the compound or the pharmaceutically acceptable salt thereof, and another therapeutic agent for HCV infectious disease being intended for combined use with;
[15] The use according to [14] above, wherein the therapeutic agent for HCV infectious disease which is for administration simultaneously or successively with another therapeutic agent for HCV infectious disease;
[16] A therapeutic method for HCV infectious disease comprising administration of the compound represented by formula (I) or the pharmaceutically acceptable salt thereof according to [1] above in need of the subject;
[17] The therapeutic method according to [16] above, wherein the compound or the pharmaceutically acceptable salt thereof is to be used in combination with another therapeutic agent for HCV infectious disease;
[18] The therapeutic method according to [17] above, wherein the compound or the pharmaceutically acceptable salt thereof and another therapeutic agent for HCV infectious disease are administered simultaneously or successively.

### Advantageous Effects of Invention

A compound represented by formula (I): 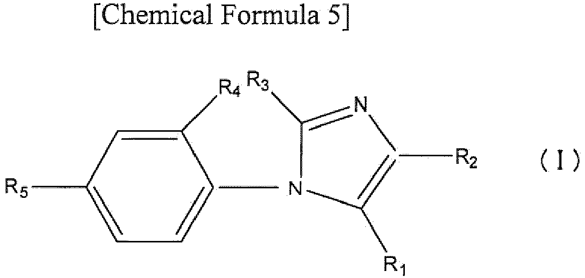 [wherein R₁, R₂, R₃, R₄ and R₅ have the same meanings as in [1] above] or a pharmaceutically acceptable salt thereof, potently inhibits HCV replication, and in particular, combination of a therapeutic agent for HCV infectious disease comprising a compound represented by formula (I) or a pharmaceutically acceptable salt thereof with another therapeutic agent for HCV infectious disease such as Interferon potently inhibits HCV replication, and is highly effective for therapy of HCV infectious disease.

### Description of Embodiments

The feature of a therapeutic agent for HCV infectious disease of the invention comprises, as an active ingredient, a compound represented by formula (I):

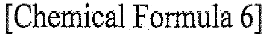

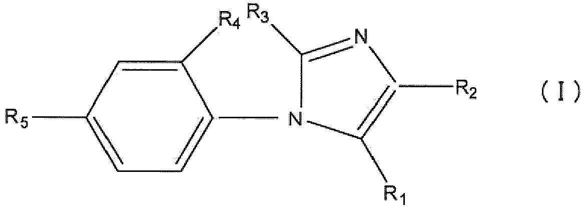

[wherein R₁ represents a hydrogen atom, a halogen atom or C₁₋₆ alkyl,
R₂ represents a hydrogen atom, C₁₋₆ alkyl or C₁₋₆ alkoxy-C₁₋₄ alkyl,
R₃ represents a hydrogen atom, C₁₋₆ alkyl or C₁₋₆ alkoxy-C₁₋₄ alkyl,
R₄ represents a hydrogen atom, a halogen atom, C₁₋₆ alkyl, C₁₋₆ alkoxy, C₃₋₆ cycloalkoxy, C₁₋₆ alkylsulfonyl, C₁₋₆ alkoxy-C₁₋₄ alkyl, hydroxy(C₁₋₆)alkyl, halo(C₁₋₆)alkyl, amino, formyl, C₂₋₄ alkanoyl, nitro or cyano, and
R₅ represents a group represented by the formula: 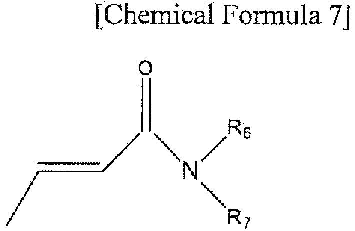 (wherein R₆ represents indanyl, chromanyl, picolyl, C₇₋₁₅ aralkyl, C₃₋₆ cycloalkyl-C₁₋₄ alkyl or N-C₇₋₁₅ aralkylamino, optionally having a substituent selected from the group consisting of halogen atom(s), C₁₋₆ alkyl, halo(C₁₋₆)alkyl, C₁₋₆ alkoxy, C₃₋₆ cycloalkylene, pyrazolyl and C₆₋₁₄ aryl,
and R₇ represents a hydrogen atom, C₁₋₆ alkyl, or hydroxy(C₁₋₆)alkyl, or R₆ and R₇, together with the nitrogen atom to which they are bonded, represent a group shown in the following table:

**[Table 3]**

| # | - N (R6)(R7) |
|---|---|
| 1 | 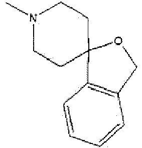 |
| 2 | 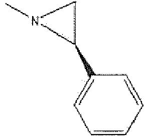 |
| 3 | 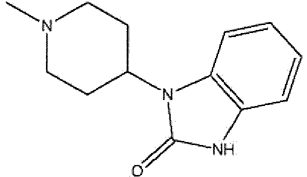 |

or a group shown in the following table:

**[Table 4]**

| # | R₅ | # | R₅ |
|---|---|---|---|
| 1 | 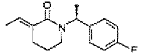 | 7 | 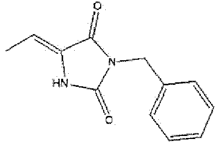 |
| 2 | 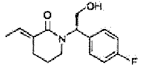 | 8 | 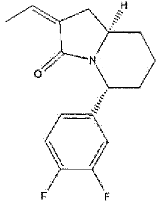 |
| 3 | 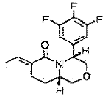 | 9 | 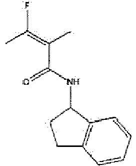 |
| 4 | 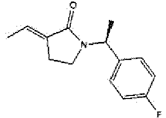 | 10 | 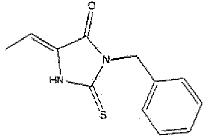 |
| 5 | 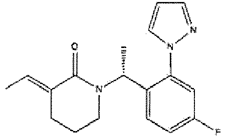 | 11 | 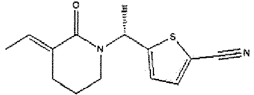 |
| 6 | 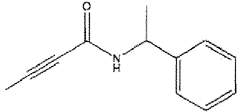 | 12 | 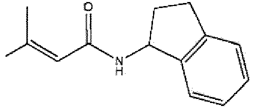 |

)] or a pharmaceutically acceptable salt thereof.

The terms and definitions used throughout the present specification will now be explained.
The term "halogen atom" refers to a fluorine atom, a chlorine atom, a bromine atom or an iodine atom. Preferred examples of "halogen atom(s)" include fluorine atom(s) and chlorine atom(s), with fluorine atom(s) being a more preferred example.
The term "C₁₋₆ alkyl" refers to a straight-chain or branched-chain alkyl, group of 1 to 6 carbons, and specific examples include methyl, ethyl, 1-propyl (n-propyl), 2-propyl (i-propyl), 2-methyl-1-propyl (i-butyl), 2-methyl-2-propyl (t-butyl), 1-butyl (n-butyl), 2-butyl (s-butyl), 1-pentyl, 2-pentyl, 3-pentyl, 2-methyl-1-butyl 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,3-dimethyl-1-butyl, 3,3-dimethyl-1-butyl, 2,2-dimethyl-1-butyl, 2-ethyl-1-butyl, 3,3-dimethyl-2-butyl and 2,3-dimethyl-2-butyl.

The term "C₁₋₆ alkoxy" refers to an oxy group bonded to "C₁₋₆ alkyl" as defined above, and specific examples include methoxy, ethoxy, 1-propyloxy, 2-propyloxy, 2-methyl-1-propyloxy, 2-methyl-2-propyloxy, 1-butyloxy, 2-butyloxy, 1-pentyloxy, 2-pentyloxy, 3-pentyloxy, 2-methyl-1-butyloxy, 3-methyl-1-butyloxy, 2-methyl-2-butyloxy, 3-methyl-2-butyloxy, 2,2-dimethyl-1-propyloxy, 1-hexyloxy, 2-hexyloxy, 3-hexyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2-methyl-3-pentyloxy, 3-methyl-3-pentyloxy, 2,3-dimethyl-1-butyloxy, 3,3-dimethyl-1-butyloxy, 2,2-dimethyl-1-butyloxy, 2-ethyl-1-butyloxy, 3,3-dimethyl-2-butyloxy and 2,3-dimethyl-2-butyloxy.
The term "C₃₋₆ cycloalkyl" refers to a monocyclic saturated aliphatic hydrocarbon group of 3 to 6 carbons, and specific examples include cyclopropyl, cyclobutyl, cyclopentyl and cyclohexyl.
The term "C₃₋₆ cycloalkoxy" refers to an oxy group bonded to "C₃₋₆ cycloalkyl" as defined above, and specific examples include cyclopropyloxy, cyclobutyloxy, cyclopentyloxy and cyclohexyloxy.

The term "C₁₋₆ alkylsulfonyl" refers to a sulfonyl group bonded to "C₁₋₆ alkyl" as defined above, and specific examples include methylsulfonyl, ethylsulfonyl, 1-propylsulfonyl, 2-propylsulfonyl, butylsulfonyl and pentylsulfonyl.
The term "C₁₋₆ alkoxy-C₁₋₄ alkyl" refers to a straight-chain or branched-chain alkyl group of 1 to 4 carbons bonded to "C₁₋₆ alkoxy" as defined above, and specific examples include methoxymethyl, ethoxymethyl, 1-propyloxymethyl, 2-propyloxymethyl, 2-methyl-1-propyloxymethyl, 2-methyl-2-propyloxymethyl, 1-butyloxymethyl, methoxyethyl, ethoxyethyl, 1-propyloxyethyl, 2-propyloxyethyl, 2-methyl-1-propyloxyethyl, 2-methyl-2-propyloxyethyl and 1-butyloxyethyl.
The term "hydroxy(C₁₋₆)alkyl" refers to "C₁₋₆ alkyl" as defined above having a hydroxy group, and specific examples include hydroxymethyl, 1-hydroxyethyl, 2-hydroxyethyl, 1-hydroxypropyl, 2-hydroxypropyl and 3-hydroxypropyl.

The term "halo(C₁₋₆)alkyl" refers to "C₁₋₆ alkyl" as defined above having halogen atom(s), and specific examples include fluoromethyl, difluoromethyl, trifluoromethyl, 1-fluoroethyl, 2-fluoroethyl, 1-fluoropropyl, 2-fluoropropyl, 3-fluoropropyl, chloromethyl, 1-chloroethyl, 2-chloroethyl, 1-chloropropyl, 2-chloropropyl, 3-chloropropyl, bromomethyl, 1-bromoethyl, 2-bromoethyl, 1-bromopropyl, 2-bromopropyl, 3-bromopropyl, iodomethyl, 1-iodoethyl, 2-iodoethyl, 1-iodopropyl, 2-iodopropyl and 3-iodopropyl. The term "C₂₋₄ alkanoyl" refers to a carbonyl group bonded to "C₁₋₆ alkyl" as defined above, and specific examples include acetyl, propionyl, isopropionyl, butyryl, isobutyryl, valeryl, isovaleryl and pivaloyl.
The term "C₃₋₆ cycloalkylene" refers to a divalent group derived by further removing any one hydrogen atom from "C₃₋₈ cycloalkyl" as defined above, and specific examples include cyclopropylene, cyclobutylene, cyclopentylene, cyclohexylene, cycloheptylene and cyclooctylene.

The term "C₆₋₁₄ aryl" refers to an aromatic hydrocarbon ring group of 6 to 14 carbons, and specific examples include phenyl and naphthyl.
The term "C₇₋₁₅ aralkyl" refers to an aromatic hydrocarbon ring group of 6 to 15 carbons formed by bonding of "C₆₋₁₄ aryl" as defined above with "C₁₋₆ alkyl" as defined above, and specific examples include benzyl, phenethyl, phenylpropyl, 1-naphthylmethyl and 2-naphthylmethyl.
The term "C₃₋₆ cycloalkyl-C₁₋₄ alkyl" refers to a group formed by bonding of "C₃₋₆ cycloalkyl" as defined above with "C₁₋₆ alkyl" as defined above, and specific examples include cyclopropylmethyl, 1-cyclopropylethyl, 2-cyclopropylethyl, 1-cyclopropylpropyl, 2-cyclopropylpropyl and 3-cyclopropylpropyl.
The term "N-C₇₋₁₅ aralkylanino" refers to a group formed by bonding of "C₇₋₁₅ aralkyl" as defined above with amino, and specific examples include N-benzylamino, N-phenethylamino, N-phenylpropylamino, N-(1-naphthylmethyl)amino and N-(2-naphthylmethyl)amino.

A compound of formula (I) will now be explained.
In formula (I), R₁, R₂, R₃, R₄ and R₅ have the following meanings.
R₁ represents a hydrogen atom, a halogen atom or C₁₋₆ alkyl, with a hydrogen atom and C₁₋₆ alkyl being preferred, and a hydrogen atom being especially preferred.
R₂ represents a hydrogen atom, C₁₋₆ alkyl or C₁₋₆ alkoxy-C₁₋₄ alkyl, with a hydrogen atom and C₁₋₆ alkyl being preferred, and a hydrogen atom being especially preferred.
R₃ represents a hydrogen atom, C₁₋₆ alkyl or C₁₋₆ alkoxy-C₁₋₄ alkyl, with a hydrogen atom and C₁₋₆ alkyl being preferred, and a hydrogen atom being especially preferred.
R₄ represents a hydrogen atom, a halogen atom, C₁₋₆ alkyl, C₁₋₆ alkoxy, C₃₋₆ cycloalkoxy, C₁₋₆ alkylsulfonyl, C₁₋₆ alkoxy-C₁₋₄ alkyl, hydroxy(C₁₋₆)alkyl, halo(C₁₋₆)alkyl, amino, formyl, C₂₋₄ alkanoyl, nitro, or cyano, among which a halogen atom, C₁₋₆ alkoxy, C₁₋₆ alkoxy-C₁₋₄ alkyl, halo(C₁₋₆)alkyl, hydroxy(C₁₋₆)alkyl and C₂₋₄ alkanoyl are preferred.
R₅ is preferably a group represented by the following formula:

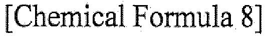

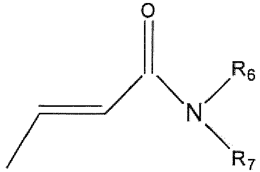

(wherein R₆ represents indanyl, chromanyl, picolyl, C₇₋₁₅ aralkyl, C₃₋₆ cycloalkyl-C₁₋₄ alkyl or N-C₇₋₁₅ aralkylamino, optionally having a substituent selected from the group consisting of halogen atom(s), C₁₋₆ alkyl, halo(C₁₋₆)alkyl, C₁₋₆ alkoxy, C₃₋₆ cycloalkylene, pyrazolyl and C₆₋₁₄ aryl,
R₇ represents a hydrogen atom, C₁₋₆ alkyl group or hydroxy(C₁₋₆) alkyl group, or R₆ and R₇, together with the nitrogen atom to which they are bonded, form a group shown in the following table:

**[Table 5]**

| # | - N (R6)(R7) |
|---|---|
| 1 | 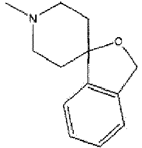 |
| 2 | 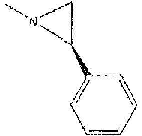 |
| 3 | 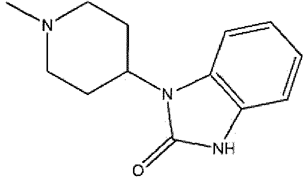 |

or a group shown in the following table:

**[Table 6]**

| # | R₅ | # | R₅ |
|---|---|---|---|
| 1 | 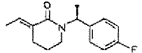 | 7 | 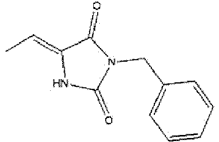 |
| 2 | 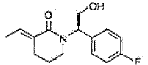 | 8 | 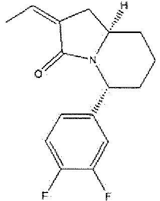 |
| 3 | 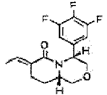 | 9 | 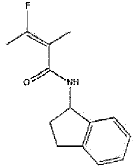 |
| 4 | 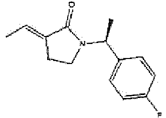 | 10 | 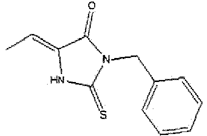 |
| 5 | 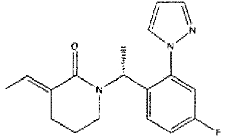 | 11 | 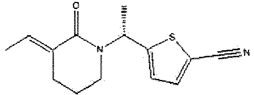 |
| 6 | 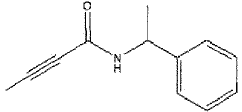 | 12 | 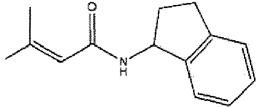 |

) and more preferably a group represented by the following formula: 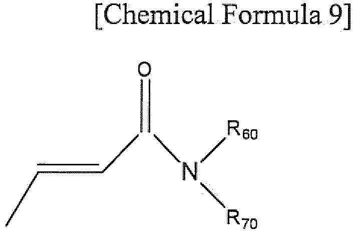 (wherein R₆₀ represents indanyl, chromanyl, picolyl, C₇₋₁₅ aralkyl, C₃₋₆ cycloalkyl-C₁₋₄ alkyl or N-C₇₋₁₅ aralkylamino, optionally having a substituent selected from the group consisting of halogen atom(s), C₁₋₆ alkyl, halo(C₁₋₆)alkyl, C₁₋₆ alkoxy, C₃₋₆ cycloalkylene, pyrazolyl and C₆₋₁₄ aryl, and
R₇₀ represents a hydrogen atom, C₁₋₆ alkyl, or hydroxy(C₁₋₆)alkyl),
or a group shown in the following table:

**[Table 7]**

| # | R₅ | # | R₅ |
|---|---|---|---|
| 1 | 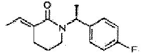 | 7 | 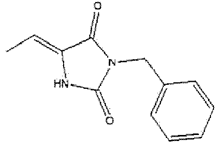 |
| 2 | 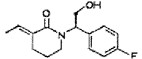 | 8 | 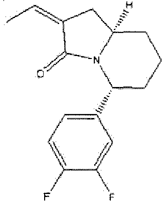 |
| 3 | 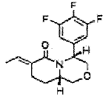 | 9 | 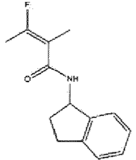 |
| 4 | 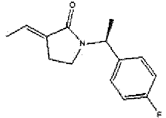 | 10 | 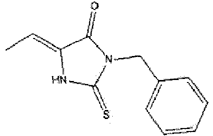 |
| 5 | 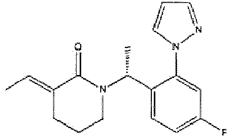 | 11 | 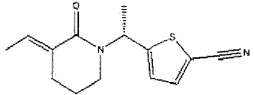 |
| 6 | 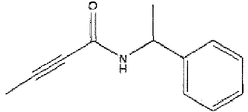 | 12 | 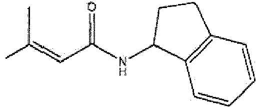 |

).

Therefore, among a compound of formula (I), a preferred compound is a compound represented by formula (I-A): 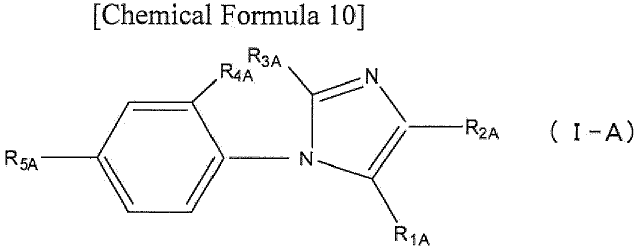 [wherein R_{1A}, R_{2A,} R_{3A} and R_{4A} have the same meanings as R₁, R₂, R₃ and R₄, respectively, and R_{5A} represents the following formula: 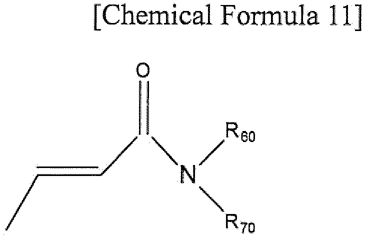 (wherein R₆₀ represents indanyl, chromanyl, picolyl, C₇₋₁₅ aralkyl, C₃₋₆ cycloalkyl-C₁₋₄ alkyl or N-C₇₋₁₅ aralkylamino, optionally having a substituent selected from the group consisting of halogen atom(s), C₁₋₆ alkyl, halo(C₁₋₆)alkyl, C₁₋₆ alkoxy, C₃₋₆ cycloalkylene, pyrazolyl and C₆₋₁₄ aryl, and
R₇₀ represents a hydrogen atom, C₁₋₆alkyl or hydroxy(C₁₋₆)alkyl)
or a formula shown in the following table:

**[Table 8]**

| # | R₅ | # | R₅ |
|---|---|---|---|
| 1 | 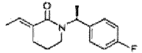 | 7 | 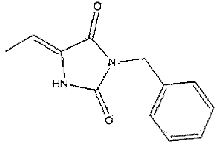 |
| 2 | 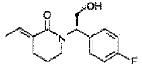 | 8 | 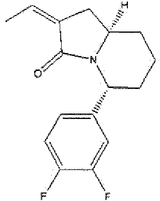 |
| 3 | 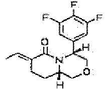 | 9 | 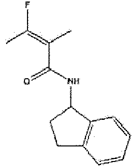 |
| 4 | 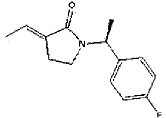 | 10 | 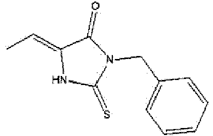 |
| 5 | 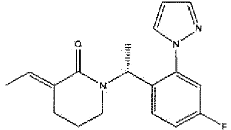 | 11 | 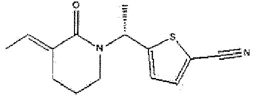 |
| 6 | 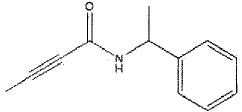 | 12 | 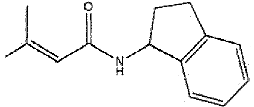 |

)] or a pharmaceutically acceptable salts thereof.

The compounds listed below, or the pharmaceutically acceptable salts, are compounds with specific excellent inhibitory activities on HCV replication.
(E)-1-[(1S)-1-(4-fluorophenyl)ethyl]-3-[3-methoxy-4-(4-methyl-1H-imi dazol-1-yl)benzylidene]piperidin-2-one (Example 425 of WO2005/115990),
(E)-1-[(1R)-1-(4-fluorophenyl)-2-hydroxyethyl]-3-[3-methoxy-4-(4-met hyl-1H-imidazol-l-yl)benzylidene]piperidin-2-one (Example 629 of WO2005/115990),
(E)-(4R,9aS)-7-[3-methoxy-4-(4-methyl-1H-imidazol-1-yl)benzylidene] -4-(3,4,5-trifluorophenyl)hexahydropyrido[2,11-c][1,4]oxazin-6-one (Example 83 of WO2007/060821),
3- [3-methoxy-4-(4-methyl-1H-imidazol-1-yl)henzylidene]-1--[(1S)-1-ph enylethyl]piperidin-2-one (Example 196 of WO2005/115990),
(E)-N-cyclohexylmethyl-3-[4-(1H-imidazol-1-yl)-3-methoxyphenyl]acr ylamide (Example 236 of WO2005/115990),
(E)-N-[1-cyclopropyl-1-phenyl]-3-[3-methoxy-4-(1H-imidazol-1-yl)phe nyl]acrylamide (Example 256 of W02005/115990),
(E)-3-[4-(1H-imidazol-1-yl)-3-trifluorophenyl]-N-indan-1-yl-acrylamid e (Example 13 of WO2005/115990),
(E)-3-[3-acetyl-4-(1H-imidazol-1-yl)phenyl]-N-indan-1-yl-acrylamide (Example 441 of WO2005/115990),
(E)-3-[3-fluoro-4-(1H-imidazol-1-yl)phenyl]-2-butenoic acid indan-1-yl-amide (Example 93 of WO2005/115990),
(E)-3-[4-(1H-imidazol-l-yl)-3-methoxyphenyl]-N-(1,2,3,4-tetrahydrona phthalen-1-yl)acrylamide (Example 231 of WO2005/115990), and
(E)-3-[4-(1H-imidazol-l-yl)-3-methoxymethylphenyl]-N-indan-1-yl-acr ylamide (Example 442 of WO2005/115990).

A compound of formula (I) of the invention can be converted to a pharmaceutically acceptable salt thereof by ordinary methods, and the pharmaceutically acceptable salts of a compound of formula (I) may be used as an active ingredient. Examples of such a salt include, specifically, an inorganic acid salt such as a sulfuric acid salt, a nitric acid salt, a perchloric acid salt, a phosphoric acid salt, a carbonate, a bicarbonic acid salt, a hydrofluoride, a hydrochloride, a hydrobromide and a hydroiodide; an organic carboxylic acid salt such as an acetic acid salt, an oxalic acid salt, a maleic acid salt, a tartaric acid salt, a fumaric acid salt and a citric acid salt; an organic sulfonic acid salt such as a methanesulfonic acid salt, a trifluoromethanesulfonic acid salt, an ethanesulfonic acid salt, a benzenesulfonic acid salt, a toluenesulfonic acid salt and a camphorsulfonic acid salt; and an amino acid salt such as an aspartic acid salt and a glutamic acid salt.

A compound of formula (I) of the invention and a pharmaceutically acceptable salt thereof may also be converted to a solvate thereof by ordinary methods. Examples of such a solvate include a hydrate, and an alcoholate such as a 1-propanolate. A compound of formula (I) and a pharmaceutically acceptable salt thereof or a solvate thereof may also be converted to amorphous or various crystalline forms by ordinary methods. Specifically, such a pharmaceutically acceptable salt, an amorphous form and a various crystalline form are described in International Patent Publication No. WO2007/058304, International Patent Publication No. WO2006/046575, International Patent Publication No. WO2009/096349 and elsewhere, and these may be employed. However, there is no particular restriction to the above.

A compound of formula (I) is within the scope of the compounds mentioned in the claims of WO2005/115990 (Patent document 3) or WO2007/060821 (Patent document 4), and most of the compounds found to have pharmacological effects in the test examples of the present invention are specifically mentioned in the examples of these international patent documents. Compounds not mentioned in the examples, apart from the explanations in the reference examples, can also be produced according to the methods described in the aforementioned international patent documents, using known compounds or commercially available compounds as starting materials.

As results of examining the inhibitory-effect on HCV replication and examining cytotoxicity of a compound of formula (I) of the invention, by using HCV subgenomic replicon cells, it has been demonstrated that a compound of formula (I) showed potent inhibitory-effect on HCV replication without cytotoxicity. In addition, it has been demonstrated that in combination with other therapeutic agents for HCV infectious disease, eg., Interferon-α-2b, a compound of formula (I) of the invention showed potent inhibitory-effect on HCV replication with very low cytotoxicity with such combined use. Thus, a compound of formula (I) and a pharmaceutically acceptable salt thereof is useful as a therapeutic agent for HCV infectious disease.

According to the invention, HCV infectious disease includes, for example, hepatitis C, and also hepatic cirrhosis, hepatic fibrosis and hepatic cancer resulting from HCV infection. Therefore, the word of therapy for HCV infectious disease means to annihilate or reduce HCV levels, and cure or alleviate symptoms of HCV infectious disease by administering a desired drug to an HCV infected patient. Also, a compound of formula (I) or a pharmaceutically acceptable salt thereof may be used for prevention of HCV infectious disease, by administration before HCV infection to prevent HCV infection, or to suppress proliferation of HCV after HCV infection in order to prevent progression to hepatic cirrhosis, hepatic fibrosis, hepatic cancer.

According to the invention, a compound of formula (I) and a pharmaceutically acceptable salt thereof may be used alone or together with another therapeutic agent for HCV infectious disease, for therapy of HCV infectious disease. The other therapeutic agent for HCV infectious disease to be used in combination therewith is preferably Interferon. The Interferon may be either Interferon-α, β or γ, and not only a natural form but also Pegylated Interferon, and a gene recombinant Interferon such as consensus Interferon. In addition, there may be used mutants, fusion proteins and fragments of these natural and gene recombinant forms of Interferon, so long as they retain the original Interferon activity. Interferon-α and β are particularly preferred for the present invention, with Interferon alfa-2b (CAS registry No. 98530-12-2) and Peginterferon alfa-2b (CAS registry No. 215647-85-1) being preferred examples.
These Interferons to be used for the invention may be produced by genetic engineering methods, or they may be purchased as commercially available products.

Other therapeutic agents for HCV infectious disease that may be used in combination with a compound of formula (I) or a pharmaceutically acceptable salt thereof include an antiviral agent such as nucleic acid analogues. A more specific example of an antiviral agent such as a nucleic acid analogue is Ribavirin (CAS Registry No. 36791-04-5). Ribavirin may be produced by the method described in US Patent No. 3,798,209, or a commercially available product may be purchased for use.
Other examples of therapeutic agents for HCV infectious disease that may be used in combination with a compound of formula (I) or a pharmaceutically acceptable salt thereof include a protease inhibitor that inhibits the activity of enzymes that act on NS proteins such as NS3, NS5A, NS4B, NS5B, which are necessary for replication of the HCV genome; and a polymerase inhibitor that inhibits RNA polymerase which is necessary for transcription of HCV More specifically, a preferred protease inhibitor includes a protease inhibitor such as Telaprevir (CAS Registry No. 402957-28-2), Boceprevir (CAS Registry No. 394730-60-0), Danoprevir (CAS Registry No. 916826-48-7) and TMC435350 (CAS Registry No. 923604-59-5), and a preferred polymerase inhibitor includes a polymerase inhibitor such as R1626 and R7128 (see WO2007/065829), Filibuvir (CAS Registry No. 877130-29-5), and MK0608 (CAS Registry No. 443642-29-3) (see NATURE REVIEWS DRUG DISCOVERY VOL.7, OCT, 2008, P799-800).
The other therapeutic agent for HCV infectious disease to be used in combination with a compound of formula (I) or a pharmaceutically acceptable salt thereof may also consist of two or more of the aforementioned drugs. For example, preferably Interferon and Ribavirin are used, and more preferably Interferon-α-2b and Ribavirin are used.

According to the invention, an example of a drug that may be used in combination with a compound of formula (I) or a pharmaceutically acceptable salt thereof, in addition to the aforementioned therapeutic agent for HCV infection, includes an antiviral agent, an anti-inflammatory agent and an immunoenhancer that may be used for prevention or therapy of hepatitis C, hepatic cirrhosis, hepatic fibrosis, hepatic cancer or the like resulting from HCV infection.

The dosage of a compound of formula (I) or a pharmaceutically acceptable salt thereof according to the invention may vary depending on the severity of symptoms, age, gender and body weight of the patient to be administered, the dosage form, or the type of salt, but will usually be about 30 µg to 10 g, preferably 100 µg to 5 g and even more preferably 100 µg to 100 mg for oral administration and 30 µg to 1 g, preferably 100 µg to 500 mg and even more preferably 100 µg to 30 mg for administration by injection, per day for adults, either once or several times in divided doses.
When a compound of formula (I) or a pharmaceutically acceptable salt thereof is to be used in combination with another therapeutic agent for HCV infectious disease, the compound of formula (I) or the pharmaceutically acceptable salt thereof according to the invention may be administered simultaneously with the other therapeutic agent for HCV infectious disease, or the compound of formula (I) or the pharmaceutically acceptable salt thereof and the other therapeutic agent for HCV infectious disease may be administered successively with an interval between them. The method of administration, interval between administrations and order of administration may be arbitrarily decided upon based on the severity of symptoms, age, gender and body weight of the patient being administered, and the dosage form. Also, the dosage of a compound of formula (I) or a pharmaceutically acceptable salt thereof according to the invention and the dosage of another therapeutic agent for HCV infectious disease will usually be about 30 µg to 10 g, preferably 100 µg to 5 g and even more preferably 100 µg to 100 mg for oral administration and 30 µg to 1 g, preferably 100 µg to 500 mg and even more preferably 100 µg to 30 mg for administration by injection, per day for adults, either once or several times in divided doses, which may be decided upon based on the situation, similar to when a compound of formula (I) or a pharmaceutically acceptable salt thereof is used alone. When a compound of formula (I) or a pharmaceutically acceptable salt thereof is to be used in combination with another therapeutic agent for HCV infectious disease, it is possible to reduce the dosages, for an advantageous effect of alleviating side-effects, because an effect beyond the additive effect due to the combined effects of their usage alone can be achieved.

A compound of formula (I) or a pharmaceutically acceptable salt thereof according to the invention, and another therapeutic agent for HCV infectious disease, may be formulated by an ordinary method. The dosage form may be, for example, as an oral drug (tablet, granules, powder, capsule, syrup or the like), an injection (for intravenous administration, for intramuscular administration, for subcutaneous administration, for intraperitoneal administration, or the like), or an external preparation (transdermal absorption preparation (ointment, medical patch or the like), eye drops, nasal drops, suppository or the like).
Solid dosage forms such as tablets, capsules, granules or powders may usually contain a compound of formula (I) or a pharmaceutically acceptable salt thereof at 0.001 to 99.5 mass% and preferably 0.001 to 90 mass%.

For production of a solid oral formulation, an excipient, binder, disintegrator, lubricant, coloring agent, taste corrective, antioxidant, dissolving aid or the like may be added as the case requires to a compound of formula (I) or a pharmaceutically acceptable salt thereof, and tablets, granules, powder or capsules prepared by an ordinary method. Also, if necessary, stabilizers, emulsifiers, absorption accelerators, surfactants and the like may be used, and tablets, granules, powders or capsules may also be subjected to film coating if necessary. Examples of excipients include lactose, saccharose, glucose, corn starch, mannitol, sorbitol, starch, pregelatinized starch, dextrin, crystalline cellulose, light silicic anhydride, aluminum silicate, calcium silicate, magnesium aluminate metasilicate and calcium hydrogenphosphate.
Examples of binders include polyvinylpyrrolidone, ethyl cellulose, methyl cellulose, gum arabic, hydroxypropyl cellulose, hydroxypropyl methyl cellulose, carboxymethyl cellulose sodium and polyvinyl alcohol.
Examples of disintegrators include crystalline cellulose, agar, gelatin, calcium carbonate, sodium hydrogencarbonate, calcium citrate, dextrin, pectin, low-substituted hydroxypropyl cellulose, carboxymethyl cellulose, carboxymethyl cellulose calcium, croscarmellose sodium, carboxymethyl starch and carboxymethyl starch sodium.
Examples of lubricants include magnesium stearate, calcium stearate, sodium stearyl fumarate and talc, examples of coloring agents include iron sesquioxide, yellow iron sesquioxide, cochineal extract, caramel, β-carotene, titanium oxide, talc; riboflavin sodium phosphate and yellow aluminum lake, and examples of taste correctives include cocoa powder, peppermint oil and cinnamon powder.

Examples of antioxidants include ascorbic acid, α-tocopherol, ethoxyquin, dibutylhydroxytoluene and butylhydroxyanisole, and examples of dissolving aids include polyethylene glycol, propylene glycol, benzyl benzoate, ethanol, cholesterol, triethanolamine, sodium carbonate, sodium citrate, polysorbate 80 and nicotinic acid amide. Examples of stabilizers include acids, bases and their salts, and examples of emulsifiers, absorption accelerators and surfactants include stearyltriethanolamine, sodium lauryl sulfate, laurylaminopropionic acid, lecithin, glycerin monostearate, sucrose fatty acid ester and glycerin fatty acid ester.
Examples of film coating agents include hydroxypropyl cellulose, hydroxypropyl methyl cellulose, ethyl cellulose and methyl cellulose. There is, of course, no limitation to the additives mentioned above.

For production of an injection (for intravenous administration, intramuscular administration, subcutaneous administration or intraperitoneal administration, for example), a pH regulator, buffering agent, suspending agent, dissolving aid, antioxidant, preservative (antiseptic agent), isotonizing agent or the like may be added to a compound of formula (I) or a pharmaceutically acceptable salt thereof as the case requires, and production carried out by an ordinary method. It may also be freeze-dried as a freeze-dried preparation to be dissolved at the time of use. Such injections may be administered into the vein, under the skin, or into the muscle.
Examples of pH regulators and buffering agents include organic acids or inorganic acids and/or their salts, sodium hydroxide, meglumine, examples of suspending agents include methyl cellulose, polysorbate 80, hydroxyethyl cellulose, gum arabic, carboxymethyl cellulose sodium and polyoxyethylene sorbitan monolaurate, examples of dissolving aids include polyoxyethylene hydrogenated castor oil, polysorbate 80, nicotinic acid amide and polyoxyethylene sorbitan monolaurate, examples of antioxidants include ascorbic acid, α-tocopherol and sulfurous acid salts, examples of preservatives include methyl paraoxybenzoate, ethyl paraoxybenzoate and sorbic acid, and examples of isotonizing agents include glucose, sodium chloride, mannitol and sorbitol, naturally with no particularly limitation to these. Such injections may usually contain the compound of formula (I) or the pharmaceutically acceptable salt thereof as 0.000001 to 99.5 mass% and preferably 0.0000001 to 90 mass%.

For production of an external preparation, a base starting material may be added to a compound of formula (I) or a pharmaceutically acceptable salt thereof, and if necessary any of the aforementioned emulsifiers, preservatives, stabilizers, pH regulators, antioxidants or coloring agents added to produce, for example, a transdermal absorption preparation (ointment, medical patch or the like), eye drops, nasal drops or suppository, by an ordinary method.
Specifically, the starting materials to be used as base starting materials may be any of various commonly employed starting materials for drugs, quasi drugs, cosmetics and the like. Specific examples include starting materials such as animal or vegetable oils, mineral oils, ester oils, waxes, fatty alcohols, fatty acids, silicon oils, surfactants, phospholipids, alcohols, polyhydric alcohols, water-soluble polymers, clay minerals, purified water.
If necessary, components with differentiation-inducing effects, such as blood flow accelerators, microbicides, antiphlogistics, cytotonic agents, vitamins, amino acids, humectants, keratolytic drugs, may also be added.
Such external preparations may usually contain the compound of formula (I) or the pharmaceutically acceptable salt thereof as 0.000001 to 99.5 mass% and preferably 0.0000001 to 90 mass%.

When a compound of formula (I) or a pharmaceutically acceptable salt thereof according to the invention is to be used in combination with another therapeutic agent for HCV infectious disease, the other therapeutic agent for HCV infectious disease may be added with it in any of the aforementioned formulations, or a formulation comprising the other therapeutic agent for HCV infectious disease alone may be prepared in the same manner as any of the formulations mentioned above, depending on the properties of the drug and the dosage form, including the method of administration. A kit may also be prepared comprising a compound of formula (I) or a pharmaceutically acceptable salt thereof according to the invention, and another therapeutic agent for HCV infectious disease.

### Examples

The invention will now be described in greater detail by examples, test examples, and reference examples, with the understanding that the invention is not limited to the examples.

The compound of Example 425 of WO2005/115990 (hereunder referred to as "bulk drug") was used to produce a 1 mg formulation, a 10 mg formulation and a 100 mg formulation (Examples 1 to 3).

### Example 1

### Preparation of 1 mg formulation

After weighing out 2 g of citric acid into a 125 mL high-density polyethylene bottle (HDPE bottle, product of HighNalge Nunc International), 18 g of purified water was added to dissolve it and form a 10% citric acid aqueous solution, while 2 g of citric acid was weighed out into another HDPE bottle, and 98 g of purified water was added to dissolve it and form a 2% citric acid aqueous solution. Separately, 100 mg of the bulk drug was weighed out into an HDPE bottle. To the HDPE bottle containing the bulk drug, 20 g of the previously prepared 10% citric acid aqueous solution was added, and after shaking to dissolve the bulk trug, 80 g of water was further added, to prepare a 2% citric acid aqueous solution containing 1 mg/g of bulk drug. Next, 1 g of the 2% citric acid aqueous solution containing 1 mg/g of bulk drug was weighed out into a separate HDPE bottle, and 9 g of 2% citric acid solution was added and mixed therewith to prepare 10 g of a 2% citric acid aqueous solution containing 0.1 mg/g of bulk drug, thereby obtaining 10 g of an oral liquid drug containing 1 mg of the bulk drug.

### Example 2

### Preparation of 10 mg formulation

A 1000 mg of bulk drug was weighed out into an HDPE bottle. To the HDPE bottle containing the bulk drug was added 20 g of the 10% citric acid aqueous solution prepared in Example 1, and after shaking to dissolve the bulk drug, 80 g of water was further added, to prepare 100 g of a 2% citric acid aqueous solution containing 10 mg/g of bulk drug. Next, 1 g of the 2% citric acid aqueous solution containing 10 mg/g of bulk drug was weighed out into a separate HDPE bottle, and 99 g of the 2% citric acid solution prepared in Example 1 was added and mixed therewith to prepare 100 g of a 2% citric acid aqueous solution containing 0.1 mg/g of bulk drug, thereby obtaining 100 g of an oral liquid drug containing 10 mg of the bulk drug.

### Example 3

### Preparation of 100 mg formulation,

A 400 mg of bulk drug was weighed out into an HDPE bottle. To the HDPE bottle containing the bulk drug was added 20 g of the 10% citric acid aqueous solution prepared in Example 1, and after shaking to dissolve the bulk drug, 80 g of water was further added, to prepare 100 g of a 2% citric acid aqueous solution containing 4 mg/g of bulk drug. Next, 25 g of the 2% citric acid aqueous solution containing 4 mg/g of bulk drug was weighed out into a separate HDPE bottle, and 75 g of the 2% citric acid solution prepared in Example 1 was added and mixed therewith to prepare 100 g of a 2% citric acid aqueous solution containing 1 mg/g of bulk drug, thereby obtaining 100 g of an oral liquid drug containing 100 mg of the bulk drug.

### Test Example 1

### Evaluation of inhibitory-activity of a Test compound on HCV replication, and cytotoxicity

The inhibitory-effect on HCV replication and the cytotoxicity of a compound of formula (I) of the invention was examined by a replicon assay using HCV subgenome replicon cells.

### 1. Method

The HCV subgenome replicon cells used were luc-ubi-neo/ET replicon cells, having the genotype Ib HCV auto-replicating subgenomic replicon, a luciferase reporter, ubiquitin, a neomycin phosphotransferase coding sequence, and three mutations for cell culturing (Pietschmann, T. et al., J. Virol. 76:4008-4021, 2002). The ET replicon cells were cultured in DMEM containing 10% FBS, 1% penicillin/streptomycin, 1% glutamine, and 250 µg/mL G418 in an incubator at 37°C, 5% CO₂. For culturing with a Test compound of formula (I), it was performed under the same conditions in DMEM containing 5% FBS, 1% penicillin/streptomycin and 1% glutamine.
The ET replicon cells were cultured in two 96-well plates, at 5000 cell/well, separately. On the following day, the Test compound alone diluting to different concentrations with a maximum concentration of 1.1 µM, was added to one plate, while human recombinant Interferon-α-2b (rIFNα-2b) at a fixed concentration of 0.1 IU/mL was added to another plate with the Test compound, simultaneously. At 72 hours after addition, the luciferase activity indicating amplification of HCV mRNA was measured. The cytotoxicity was measured with a CytoTox- Cell Proliferation Assay (Promega Corporation).

### 2. Results

The luciferase activities, for the compound of formula (I) alone and for a combination of the compound of formula (I) and rIFNα-2b, were shown in Table 1 as percentage with respect to luciferase activity when using the solvent alone. The HCV replication inhibitory-activity was determined as the concentration which produced 50% inhibition of HCV replicon (EC₅₀), and the cytotoxicity was determined as the concentration which produced 50% reduction in viable cells (IC₅₀), while the selectivity was determined as the value of IC₅₀/EC₅₀; the results are summarized in Table 2.

[Table 9]

**Table 1: Luciferase activities of a Test compound**

| Concentration of Test compound A or Test compound B | Test compound A | Test compound A + rIFNα-2b | Test compound B | Test compound B + rIFNα-2b |
|---|---|---|---|---|
| 0.003 µM | 89% | 73% | 73% | 77% |
| 0.001 µM | 87% | 71% | 72% | 71% |
| 0.03 µM | 87% | 75% | 62% | 69% |
| 0.11 µM | 89% | 65% | 68% | 61% |
| 0.35 µM | 51% | 40% | 48% | 41% |
| 1.1 µM | 21% | 12% | 30% | 18% |

| | | | | |
|---|---|---|---|---|
| Test compound A: Compound of Example 425 of WO2005/115990 Test compound B: Compound of Example 83 of WO2007/060821 | | | | |

[Table 10]

**Table 2: Evaluation results by replicon assay method**

| Test compound | Maximum concentration of Test compound | HCV replication inhibitory activity EC₅₀ | Cytotoxicity IC₅₀ | Selectivity IC₅₀/EC₅₀ |
|---|---|---|---|---|
| A | 1.1 µM | 0.36 µM | >1.1 µM | >3.06 |
| A + | 1.1 µM | 0.22 µM | >1.1 µM | >5 |
| rIFNα-2b | | | | |
| B | 1.1 µM | 0.31 µM | >1.1 µM | >3.55 |
| B + | 1.1 µM | 0.21 µM | >1.1 µM | >5.24 |
| rIFNα-2b | | | | |

| | | | | |
|---|---|---|---|---|
| Test compound A: Compound of Example 425 of W02005/115990 Test compound B: Compound of Example 83 of WO2007/060821 | | | | |

As clearly seen by the results in Table 1, when Test compound A and Test compound B were each used alone, luciferase activity decreased in approximately a dose-dependent manner with the increase of concentration, and therefore Test compound A and Test compound B each exhibited approximately dose-dependent HCV replication inhibitory-activity. Also, when Test compound A and Test compound B were used in combination with rIFNα-2b, each showed more potent reduction of HCV replication with dose-dependent manner.
As clearly seen by the results in Table 2, when Test compound A and Test compound B were each used alone, they exhibited potent HCV replication inhibitory-activity at lower concentrations than the concentrations for the cytotoxicity. Also, when Test compound A and Test compound B were each used in combination with rIFNα-2b, they showed even more potent HCV replication inhibitory-activity at lower concentrations than the concentrations for the cytotoxicity.
Thus, a compound of formula (I) or a pharmaceutically acceptable salt thereof exhibited HCV replication inhibitory-effects and reduced cytotoxicity.

### Test Example 2

### Evaluation of inhibitory-activity of a Test compound on HCV replication, and cytotoxicity

The inhibitory-effect on HCV replication and the cytotoxicity of a compound of formula (I) of the invention were examined by a replicon assay method using different HCV subgenomic replicon cells from Example 1, specifically LucNeo#2 replicon cells. The LucNeo#2 replicon cells are reporter cells created with the same concept as the luc-ubi-neo/ET replicon cells used in Test Example 1, and are utilized for analysis of the RNA replication mechanism of HCV or anti-HCV drug evaluation, but the gene sequence differs from that of luc-ubi-neo/ET replicon cells (Biochem. Biophys. Res. Commun. 343 (2006) 879-884).

### 1. Method

LucNeo#2 replicon cells cultured to confluency were recovered by trypsin treatment, and were spread on a collagen-coated plate at a density of 20,000 cells/mL. The cell culture was conducted in an incubator at 37°C, 5% CO₂, with 10% FCS/DNEM (containing mixed solution of penicillin-streptomycin, G418 and a non-essential amino acid). After 24 hours, the cells were rinsed and a compound of formula (I) was added as Test compound to final concentrations of 3.3 µM or 10 µM. At 72 hours after addition of the Test compound, the cells of one plate were rinsed, the cells were lysed with M-PER, and the intracellular luciferase activity was measured using a Promega kit. An Alamar Blue solution was also added to cells cultured under the same conditions, and the cytotoxicity was examined.

### 2. Results

The intracellular luciferase activity and the Alamar Blue signal for cytotoxicity at 72 hours after Test compound addition were shown in Table 3 as a percentage with respect to the solvent-treated group.

[Table 11]

**Table 3: Luciferase activity and cytotoxicity**

| Test compound | Concentration | Luciferase activity | Cytotoxicity |
|---|---|---|---|
| A | 3.3 µM | 116.60% | 100.00% |
| A | 10 µM | 42.80% | 101.50% |
| C | 3.3 µM | 93.50% | 101.00% |
| C | 10 µM | 55.00% | 102.00% |
| B | 3.3 µM | 50.30% | 101.00% |
| B | 10 µM | 27.80% | 100.75% |

| | | | |
|---|---|---|---|
| Test compound A: Compound of Example 425 of WO2005/115990 Test compound B: Compound of Example 83 of WO2007/060821 Test compound C: Compound of Example 629 of WO2005/115990 | | | |

As clearly seen by the results in Table 3, Test compound A, Test compound B and Test compound C showed potent HCV replication inhibitory-activity by reducing the luciferase activity in a dose-dependent manner, at lower concentrations than the concentrations exhibiting cytotoxicity. Thus, a compound of formula (I) or a pharmaceutically acceptable salt thereof exhibited HCV replication inhibitory-effects and reduced cytotoxicity.

### Test Example 3

### Inhibitory-activity of a Test compound on HCV replication,

LucNeo#2 replicon cells, which were the HCV subgenomic replicon cells used in Test Example 2, were used to examine HCV replication inhibitory-activity, from which signals were corrected by using cell number.

### 1. Method

LucNeo#2 replicon cells cultured to confluency were recovered by trypsin treatment, and were spread on a collagen-coated plate at a density of 1000 cells/well. The cell culture was conducted in an incubator at 37°C, 5% CO₂, with 10% FBS/DMEM (containing mixed solution of penicillin-streptomycin and a non-essential amino acid). On the following day or the evening after confirmation of cell adhesion, a compound of formula (I) as the Test compound was added to final concentration of 1.0 to 1.3 µM. At 4 days after the Test compound addition, Alamar Blue diluted 5-fold with PBS(-) was added in an amount of 1/10 for measurement of the fluorescence, to determine the cell count. After the Alamar Blue measurement, the luminescence from intracellular luciferase activity was determined by adding substrate solution in an amount of 5/11 using a Promega kit.

### 2. Results

The Alamar Blue activity and luciferase activity were determined by subtracting the values measured for wells without cells. Next, the luciferase activity value × 1000 was divided by the value from Alamar-Blue measurement, then, the total activity was defined as the value calculated without the Test compound, and the percentages of inhibition by the Test compound was calculated in respect to total activity and listed in Table 4.

[Table 12-1]

**(Table 4-1)**

| | | | | | | | | | |
|---|---|---|---|---|---|---|---|---|---|
| 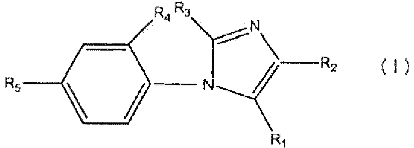 | | | | | | | | | |
| | | | | | R₅ | | | | Inhibition (%) at 1 uM |
| | | | | | 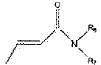 | | | | |

| # | R₁ | R₂ | R₃ | R₄ | R₆ | R₇ | Structural formula | Example No. of test compound | |
|---|---|---|---|---|---|---|---|---|---|
| 1 | H | CH₃ | CH₃ | OCH₃ | 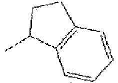 | H | 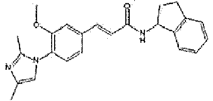 | - | 74.0 |
| 2 | H | CH₃ | H | OCH₃ | 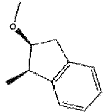 | H | 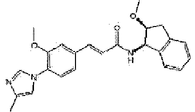 | Example 851 of W02005/115990 | 70.7 (at 1.2uM) |
| 3 | H | CH₃ | H | OCH₃ | 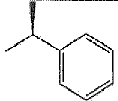 | H | 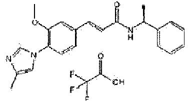 | Example 196 of WO2005/115990 | 81.7 (at 1.1uM) |

**[Table 12-2]**

| | | | | | | | | | |
|---|---|---|---|---|---|---|---|---|---|
| 4 | H | CH₃ | H | OCH₃ | 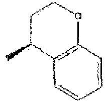 | H | 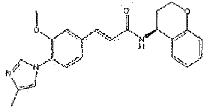 | Example 414 of WO2005/115990 | 55.5 (at 1.3uM) |
| 5 | H | CH₃ | H | OCH₃ | 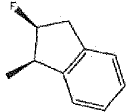 | H | 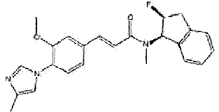 | Example 142 of WO2005/115990 | 54.6 |
| 6 | H | CH₃ | H | OCH₃ | 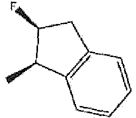 | H | 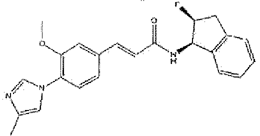 | Example 143 of WO2005/115990 | 53.3 |
| 7 | H | CH₃ | H | OCH₃ | 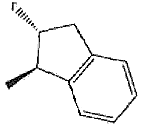 | H | 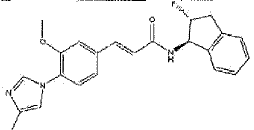 | Example 149 of WO2005/115990 | 63.8 |
| 8 | Cl | H | H | F | 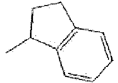 | H | 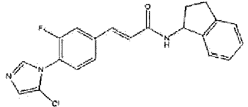 | Example 451 of WO2005/115990 | 82.5 |

**[Table 12-3]**

| | | | | | | | | | |
|---|---|---|---|---|---|---|---|---|---|
| 9 | H | H | H | NH₂ | 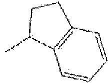 | H | 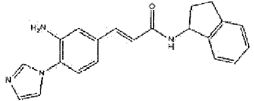 | Example 322 of WO2005/115990 | 81.3 |
| 10 | H | H | H | OCH₃ | 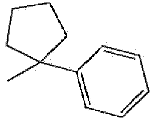 | H | 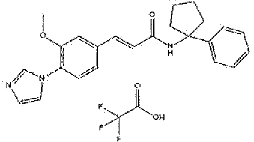 | Example 257 of WO2005/115990 | 79.5 |

**[Table 12-4]**

| | | | | | | | | | |
|---|---|---|---|---|---|---|---|---|---|
| 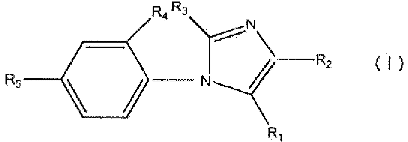 | | | | | | | | | |
| | | | | | R₅ | | | | Inhibition (%) at 1 uM |
| | | | | | 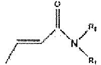 | | | | |

| # | R₁ | R₂ | R₃ | R₄ | R₅ | R₇ | Structural formula | Example No. of test compound | |
|---|---|---|---|---|---|---|---|---|---|
| 11 | H | H | H | CH₂OH | 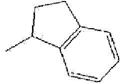 | H | 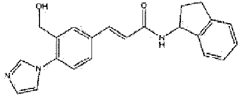 | Example 439 of WO2005/115990 | 81.5 |
| 12 | H | CH₃ | H | H | 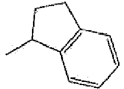 | H | 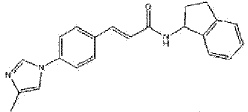 | Example 52 of WO2005/115990 | 76.6 |
| 13 | H | H | H | OCH₃ | 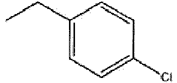 | H | 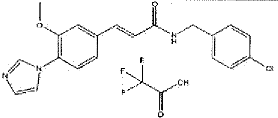 | Example 221 of WO2005/115990 | 65.9 |

**[Table 12-5]**

| | | | | | | | | | |
|---|---|---|---|---|---|---|---|---|---|
| 14 | H | H | H | CH(CH₃)OH | 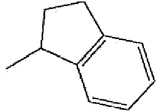 | H | 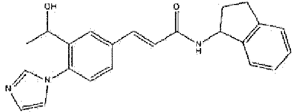 | Example 440 of WO2005/115990 | 73.4 |
| 15 | H | H | H | CHO | 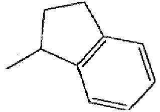 | H | 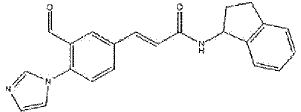 | Example 437 of WO2005/115990 | 73.3 |
| 16 | H | H | H | OCH₃ | 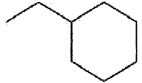 | H | 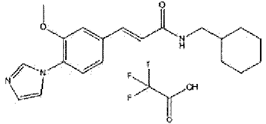 | Example 236 of WO2005/115990 | 71.2 |
| 17 | H | H | H | OCH₃ | 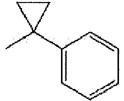 | H | 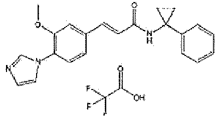 | Example 256 of WO2005/115990 | 87.7 |
| 18 | H | CH₃ | H | Br | 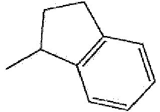 | H | 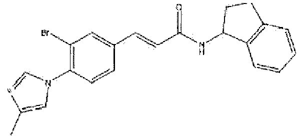 | Example 44 of WO2005/115990 | 75.3 |

**[Table 12-6]**

| | | | | | | | | | |
|---|---|---|---|---|---|---|---|---|---|
| 19 | H | CH₃ | H | Cl | 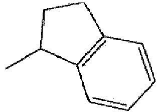 | H | 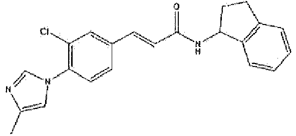 | Example 43 of WO2005/115990 | 75.4 |
| 20 | H | H | H | CF₃ | 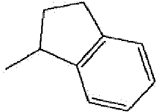 | H | 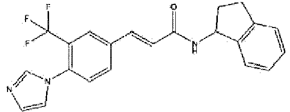 | Example 13 of WO2005/115990 | 76.7 |

**[Table 12-7]**

| | | | | | | | | | |
|---|---|---|---|---|---|---|---|---|---|
| 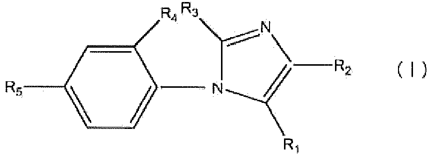 | | | | | | | | | |
| | | | | | R₅ | | | | Inhibition (%) at 1 uM |
| | | | | | 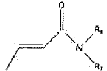 | | | | |

| # | R₁ | R₂ | R₃ | R₄ | R₆ | R₇ | Structural formula | Example No. of test compound | |
|---|---|---|---|---|---|---|---|---|---|
| 21 | H | H | H | CH₃CO | 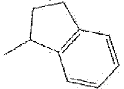 | H | 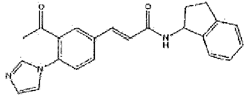 | Example 441 of WO2005/115990 | 81.9 |
| 22 | H | H | H | OCH₃ | 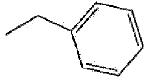 | CH₃ | 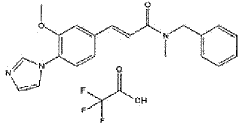 | Example 238 of WO2005/115990 | 66.3 |
| 23 | H | H | H | OCH₃ | 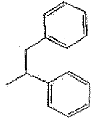 | H | 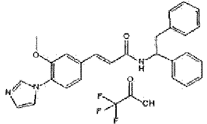 | Example 244 of WO2005/115990 | 71.6 |

**[Table 12-8]**

| | | | | | | | | | |
|---|---|---|---|---|---|---|---|---|---|
| 24 | H | H | H | NO₂ | 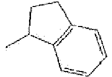 | H | 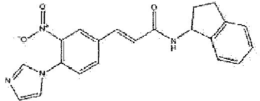 | Example 320 of WO2005/115990 | 76.7 |
| 25 | CH₃ | H | H | CN | 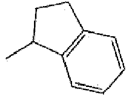 | H | 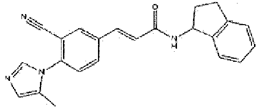 | Example 446 of WO2005/115990 | 83.5 |
| 26 | H | H | H | Cl | 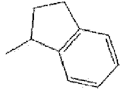 | H | 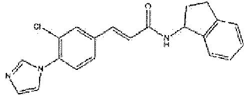 | Example 11 of WO2005/115990 | 75.5 |
| 27 | H | H | H | OCH₃ | 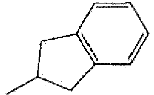 | H | 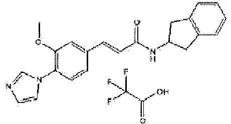 | Example 230 of WO2005/115990 | 69.0 |
| 28 | H | H | H | OCH₃ | 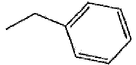 | H | 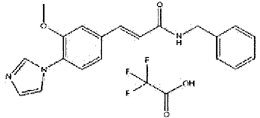 | Example 232 of WO2005/115990 | 70.5 |

**[Table 12-9]**

| | | | | | | | | | |
|---|---|---|---|---|---|---|---|---|---|
| 29 | H | H | H | OCH₃ | 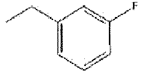 | H | 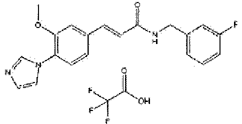 | Example 255 of WO2005/115990 | 62.8 |
| 30 | H | H | H | OCH₃ | 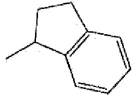 | H | 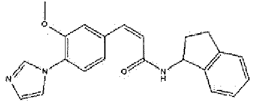 | Example 323 of WO2005/115990 | 66.4 |

**[Table 12-10]**

| | | | | | | | | | |
|---|---|---|---|---|---|---|---|---|---|
| 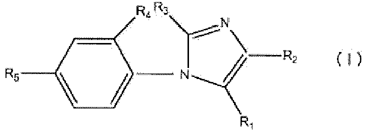 | | | | | | | | | |
| | | | | | R₅ | | | | Inhibition at 1 uM |
| | | | | | 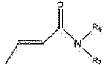 | | | | |

| # | R₁ | R₂ | R₃ | R₄ | R₆ | R₇ | Structural formula | Example No. of test compound | |
|---|---|---|---|---|---|---|---|---|---|
| 31 | H | H | H | CH₃ | 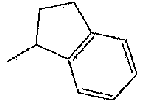 | H | 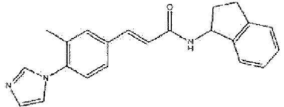 | Example 25 of WO2005/115990 | 71.0 |
| 32 | Cl | H | H | OCH₃ | 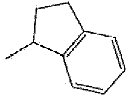 | H | 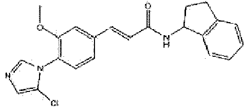 | Example 21 of WO2005/115990 | 71.8 |
| 33 | H | H | H | OCH₃ | 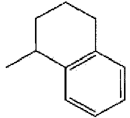 | H | 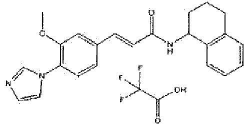 | Example 231 of WO2005/115990 | 86.8 |

**[Table 12-11]**

| | | | | | | | | | |
|---|---|---|---|---|---|---|---|---|---|
| 34 | H | H | H | CH₃SO₂ | 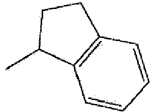 | H | 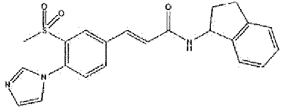 | Example 56 of WO2005/115990 | 70.2 |
| 35 | CH₃ | CH₃ | H | F | 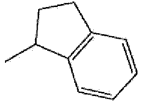 | H | 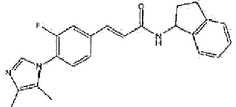 | Example 33 of WO2005/115990 | 77.2 |
| 36 | H | CH₃ | H | OCH₃ | 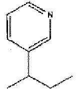 | H | 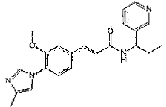 | Example 204 of WO2005/115990 | 75.7 |
| 37 | H | H | H | OCH₃ | 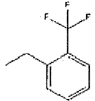 | H | 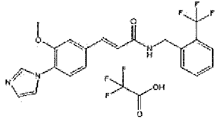 | Example 225 of WO2005/115990 | 77.1 |
| 38 | H | H | H | OCH₃ | 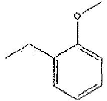 | H | 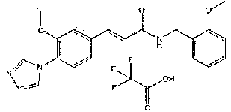 | Example 214 of WO2005/115990 | 63.7 |

**[Table 12-12]**

| | | | | | | | | | |
|---|---|---|---|---|---|---|---|---|---|
| 39 | H | H | H | OCH₃ | 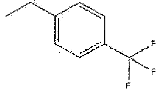 | H | 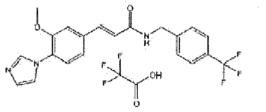 | Example 227 of WO2005/115990 | 53.2 |
| 40 | H | H | H | F | 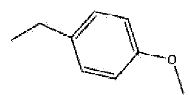 | H | 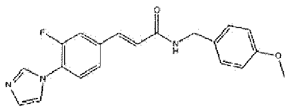 | Example 327 of WO2005/115990 | 59.4 |

**[Table 12-13]**

| | | | | | | | | | |
|---|---|---|---|---|---|---|---|---|---|
| 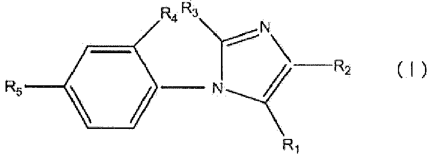 | | | | | | | | | |
| | | | | | R₅ | | | | Inhibition (%) at 1 uM |
| | | | | | 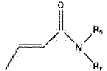 | | | | |

| # | R₁ | R₂ | R₃ | R₄ | R₆ | R₇ | Structural formula | Example No. of test compound | |
|---|---|---|---|---|---|---|---|---|---|
| 41 | H | H | H | OCH₃ | 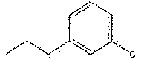 | H | 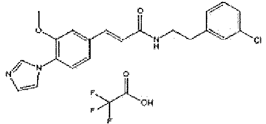 | Example 275 of WO2005/115990 | 71.8 |
| 42 | H | H | H | OCH₃ | 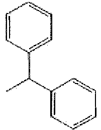 | H | 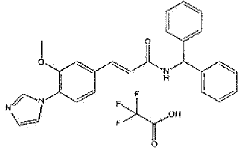 | Example 228 of WO2005/115990 | 75.8 |

**[Table 12-14]**

| | | | | | | | | | |
|---|---|---|---|---|---|---|---|---|---|
| 43 | H | CH₂OCH₃ | H | F | 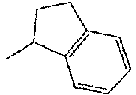 | H | 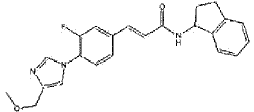 | Example 447 of WO2005/115990 | 55.8 |
| 44 | H | H | H | (CH₃)₂CHO | 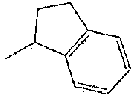 | H | 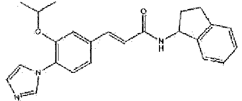 | Example 457 of WO2005/115990 | 64.6 |
| 45 | H | H | H | OCH₃ | 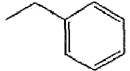 | H | 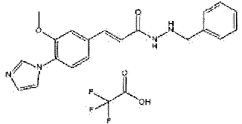 | Example 271 of WO2005/115990 | 68.8 |
| 46 | H | H | H | CH₂OCH₃ | 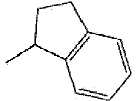 | H | 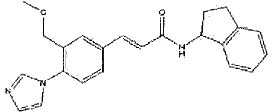 | Example 442 of WO2005/115990 | 86.4 |
| 47 | H | CH₃ | H | 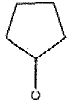 | 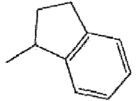 | H | 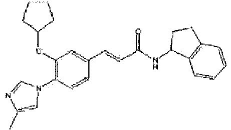 | Example 59 of WO2005/115990 | 63.8 |

**[Table 12-15]**

| | | | | | | | | | |
|---|---|---|---|---|---|---|---|---|---|
| | | | | | 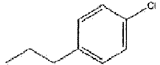 | | 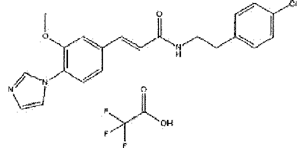 | | 61.3 |
| 48 | H | H | H | OCH₃ | | H | | Example 276 of WO2005/115990 | |
| 49 | H | CH₃ | CH₃ | OCH₃ | 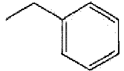 | 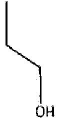 | 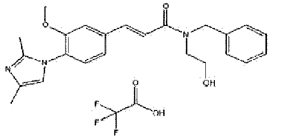 | - | 71.6 |

[Table 12-16]

**(Table 4-2)**

| | | | | | | | | |
|---|---|---|---|---|---|---|---|---|
| 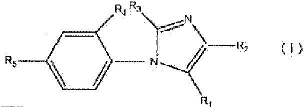 | | | | | | | | |

| # | R₁ | R₂ | R₃ | R₄ | R₅ | Structural formula | Example No. of test compound | Inhibition (%) at 1 uM |
|---|---|---|---|---|---|---|---|---|
| 50 | H | CH₃ | CH₃ | CH₃O | 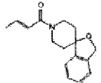 | 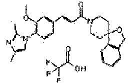 | Reference Example 1 | 52.3 |
| 51 | H | CH₃ | H | CH₃O | 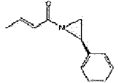 | 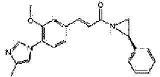 | Reference Example 2 | 62.2 |
| 52 | H | H | H | CH₃O | 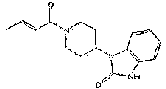 | 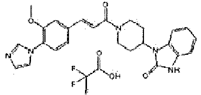 | Example 385 of WO2005/115990 | 51_{.}4 |

**[Table 12-17]**

| | | | | | | | | |
|---|---|---|---|---|---|---|---|---|
| 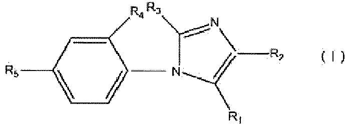 | | | | | | | | |

| # | R₁ | R₂ | R₃ | R₄ | R₅ | Structural formula | Example of test compound | Inhibition (%) at 1 uM |
|---|---|---|---|---|---|---|---|---|
| 53 | H | CH₃ | H | OCH₃ | 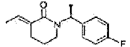 | 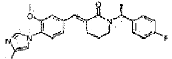 | Example 425 of WO2005/115990 | 30.9 |
| 54 | H | CH₃ | H | OCH₃ | 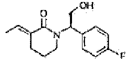 | 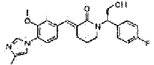 | Example 629 of WO2005/115990 | 14.2 |
| 55 | H | CH₃ | H | OCH₃ | 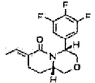 | 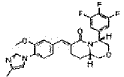 | Example 83 of WO2007/060821 | 66.8 |
| 56 | H | CH₃ | H | OCH₃ | 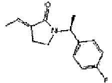 | 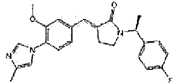 | Example 1054 of WO2005/115990 | 65.8 |
| 57 | H | CH₃ | H | OCH₃ | 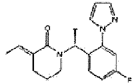 | 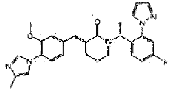 | Example 920 of WO2005/115990 | 55.8 |

**[Table 12-18]**

| | | | | | | | | |
|---|---|---|---|---|---|---|---|---|
| 58 | H | CH₃ | H | OCH₃ | 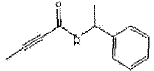 | 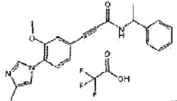 | Example 83 of WO2005/115990 | 64.6 |

**[Table 12-19]**

| | | | | | | | | |
|---|---|---|---|---|---|---|---|---|
| 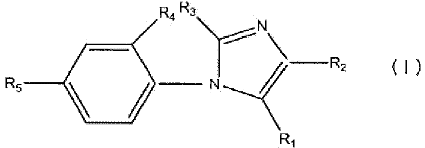 | | | | | | | | |

| # | R₁ | R₂ | R₃ | R₄ | R₅ | Structural formula | Example No. of test compound | Inhibition (%) at 1 uM |
|---|---|---|---|---|---|---|---|---|
| 59 | H | CH₃ | H | OCH₃ | 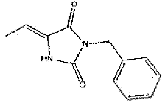 | 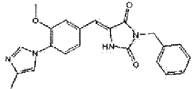 | Example 408 of WO2005/115990 | 51.9 |
| 60 | H | CH₃ | H | OCH₃ | 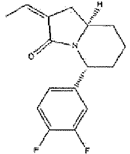 | 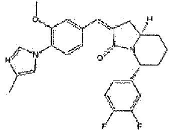 | Example 26 of WO2007/060821 | 50.5 |
| 61 | H | CH₃ | H | OCH₃ | 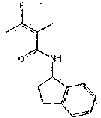 | 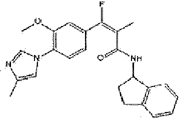 | Example 1055 of WO2005/115990 | 50.5 |
| 62 | H | CH₃ | H | OCH₃ | 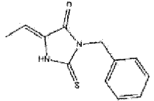 | 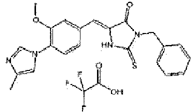 | Example 411 of WO2005/115990 | 68.2 |

**[Table 12-20]**

| | | | | | | | | |
|---|---|---|---|---|---|---|---|---|
| 63 | H | CH₃ | H | OCH₃ | 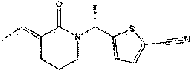 | 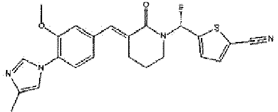 | Example 1066 of WO2005/115990 | 70.7 |
| 64 | H | H | H | F | 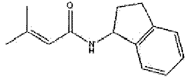 | 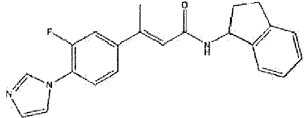 | Example 93 of WO2005/115990 | 78.0 |

As shown in Table 4, a compound of formula (I) reduced luciferase activity per cell, and exhibited potent HCV replication inhibitory activity.

### Test Example 4

### Lowering effect of a Test compound on blood HCV levels

The lowering effect on serum HCV RNA levels was measured to determine the HCV replication inhibitory-effect of a compound of formula (I) of the invention were determined by measuring the serum HCV RNA levels.

### 1. Method

Human hepatocyte-transplanted model mice (PXB mice) were prepared from uPA^{+/+}/SCID mice as host mice. The uPA^{+/+}/SCID mice are characterized by a combination of liver damage and serious immune deficiency complications. Commercially available frozen human hepatocytes (BD Biosciences, MA, USA) were grafted by infusion into the spleens of male or female uPA^{+/+}/SCID mice that had reached a postnatal age of 2 to 4 weeks, to create human hepatocyte-transplanted mice (PXB mice) having at least 70% of the mouse liver parenchymal cells replaced with human hepatocytes. After 10 to 14 weeks from transplantation, at least 7.0 mg/mL of human albumin in blood was confirmed, indicating that the graft had been established.
HCV 1b genotype-infected patient serum (PhoenixBio) was injected through the orbital socket to a virus titer of 1 × 10⁴ copies/mouse as measured by RT-PCR (Life Technologies Corporation, Carlsbad, USA), for the HCV infection. In this test, HCV infection was considered to have been established if the individual had a serum HCV RNA level of 1 × 10⁶ copies/mL, at 7 days counting backward from initial administration of the drug.
Test compound A (compound of Example 425 of WO2005/115990) suspended in a 0.5% methyl cellulose solution was used for the oral administration at doses of 30 and 100 mg/kg/10 mL. The number of mice per group was five. The solvent alone was used as a negative control. Administration was done once per day for 14 consecutive days. With the initial administration as day 0, blood was collected on day 0, 1, 3, 7, 10, 14, 17 and 21, and the serum HCV RNA levels were measured.

### 2. Results

The percent change of blood HCV RNA levels in 14 days, which was 24 hours after final dosing (13 day), from those in day 0, which was predose, was calculated for each individual. The average value and SE for each group are shown in Table 5.

[Table 13]

**Table 5: Change in serum HCV RNA levels**

| | Test compound | Test compound A (mg/kg/day) | | |
|---|---|---|---|---|
| | Vehicle | 10 | 30 | 100 |
| Average | 90.1 | 73.0 | 50.4 | 32.6 |
| S.E. | 9.1 | 17.8 | 9.2 | 6.5 |

| | | | | |
|---|---|---|---|---|
| Test compound A: Compound of Example 425 of WO2005/115990 | | | | |

As shown in Table 5, Test compound A exhibited a dose-dependent reduction in serum HCV RNA level. Thus, a compound of formula (I) or a pharmaceutically acceptable salt thereof exhibited HCV replication inhibitory-effect.

### Reference Example 1

### Synthesis of (E)-3-[4-(2,4-dimethyl-1H-imidazol-1-yl)-3-methoxyphenyl-1-{3H-spi ro[2-benzofuran-1,4-piperidine]-1'-yl}prop-2-en-1-one

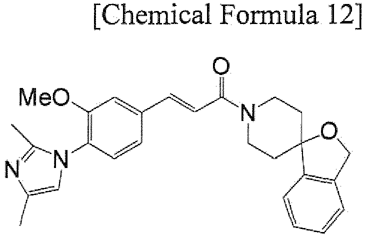

The title compound was obtained in an amount of 7.29 mg by reaction according to the synthesis method described in WO2005115990, and purification of the obtained crude product by LC-MS. The physical values of the compound are as follows.
ESI-MS; m/z 444[M++H].

### Reference Example 2

### Syntheses of (E)-3-[3-methoxy-4-(4-methul-1H-imidazol-1-yl)phenyl]-1-[(R)-2-phen ylaziridin-1-yl]prop-2-en-1-one

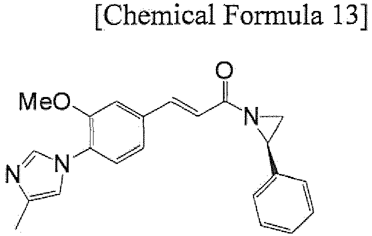

The title compound can be obtained by reaction according to the synthesis method of Example 147 of WO2005/115990. The physical values of the compound are as follows.
1H-NMR (CDCl3) δ(ppm):7.73 (s, 1H), 7.34-7.45 (m, 6H), 7.25-7.27 (m, 1H), 7.15-7.17 (m, 1H), 6.94 (s, 1H), 6.73 (d, J = 16.0 Hz, 1H), 5.59 (dd, J = 10.0, 8.0 Hz, 1H), 4.43 (dd, J = 16.0,10.0 Hz, 1H), 3.95 (dd, J = 16.0, 8.0 Hz, 1H), 3.89 (s, 3H), 2.30 (s, 3H).

### Industrial Applicability

As clearly explained in detail above, a compound of formula (I) or a pharmaceutically acceptable salt thereof inhibits HCV replication, and its use in combination with other therapeutic agents for HCV infectious disease such as Interferon can inhibit HCV replication more potently. Thus, a compound of formula (I) and a pharmaceutically acceptable salt thereof is highly effective for the prevention and therapy of HCV infectious disease.

## Claims

1. A therapeutic agent for HCV infectious disease comprising a compound represented by formula (I): 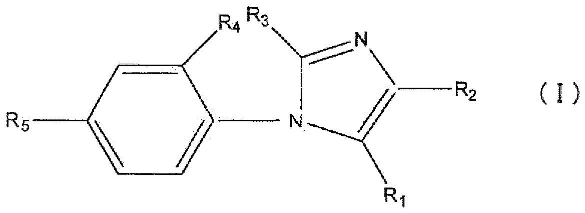 wherein R₁ represents a hydrogen atom, a halogen atom or C₁₋₆ alkyl,
R₂ represents a hydrogen atom, C₁₋₆ alkyl or C₁₋₆ alkoxy-C₁₋₄ alkyl,
R₃ represents a hydrogen atom, C₁₋₆ alkyl or C₁₋₆ alkoxy-C₁₋₄ alkyl,
R₄ represents a hydrogen atom, a halogen atom, C₁₋₆ alkyl, C₁₋₆ alkoxy, C₃₋₆ cycloalkoxy, C₁₋₆ alkylsulfonyl, C₁₋₆ alkoxy-C₁₋₄ alkyl, hydroxy(C₁₋₆)alkyl, halo(C₁₋₆)alkyl, amino, formyl, C₂₋₄ alkanoyl, nitro or cyano, and
R₅ represents a group represented by the formula: 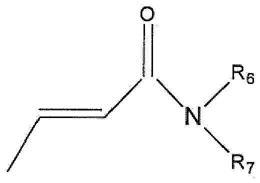 or a group shown in the following table:
| # | R₅ | # | R₅ |
|---|---|---|---|
| 1 | 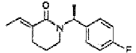 | 7 | 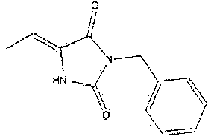 |
| 2 | 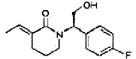 | 8 | 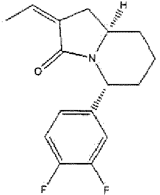 |
| 3 | 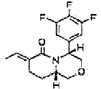 | 9 | 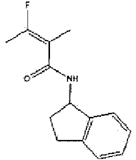 |
| 4 | 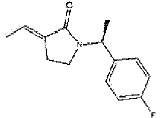 | 10 | 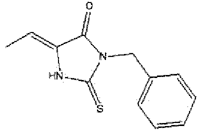 |
| 5 | 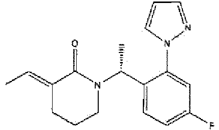 | 11 | 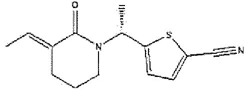 |
| 6 | 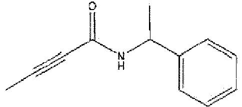 | 12 | 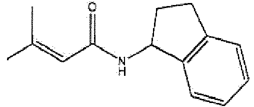 |
wherein R₆ represents indanyl, chromanyl, picolyl, C₇₋₁₅ aralkyl, C₃₋₆ cycloalkyl-C₁₋₄ alkyl or N-C₇₋₁₅ aralkylamino,
optionally having a substituent selected from the group consisting of halogen atoms, C₁₋₆ alkyl, halo(C₁₋₆)alkyl, C₁₋₆ alkoxy, C₃₋₆ cycloalkylene, pyrazolyl and C₆₋₁₄ aryl, and
R₇ represents a hydrogen atom, C₁₋₆ alkyl or hydroxy(C₁₋₆)alkyl, or R₆ and R₇, together with the nitrogen atom to which they are bonded, represent a group shown in the following table:
| # | - N (R6)(R7) |
|---|---|
| 1 | 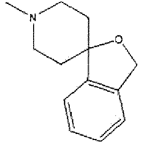 |
| 2 | 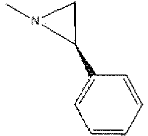 |
| 3 | 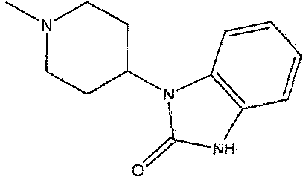 |
or a pharmaceutically acceptable salt thereof.

2. The therapeutic agent for HCV infectious disease comprising the compound represented by formula (I): 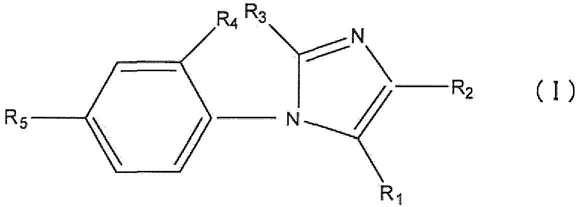 wherein R₁, R₂, R₃, R₄ and R₅ have the same meanings as in claim 1, or the pharmaceutically acceptable salt thereof according to claim 1, which is for use in combination with another therapeutic agent for HCV infectious disease.

3. The therapeutic agent for HCV infectious disease comprising the compound represented by formula (I): 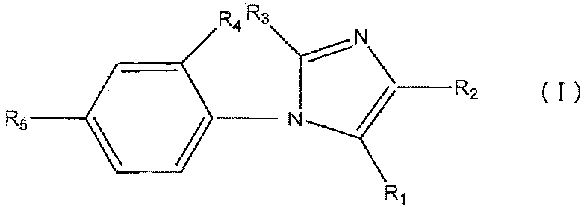 wherein R₁, R₂, R₃, R₄ and R₅ have the same meanings as in claim 1 or the pharmaceutically acceptable salt thereof according to claim 1 or 2, which is for administration either simultaneously or successively with another therapeutic agent for HCV infectious disease.

4. The therapeutic agent for HCV infectious disease comprising the compound represented by formula (I) or the pharmaceutically acceptable salt thereof, and another therapeutic agent for HCV infectious disease.

5. The therapeutic agent for HCV infectious disease according to any one of claims 1 to 5, wherein the compound represented by formula (I) or the pharmaceutically acceptable salt thereof is a compound selected from the group consisting of:
(E)-1-[(1S)-1-(4-fluorophenyl)ethyl]-3-[3-methoxy-4-(4-methyl-1H-imi dazol-1-yl)benzylidene]piperidin-2-one,
(E)-1-[(1R)-1-(4-fluorophenyl)-2-hydroxyethyl]-3-[3-methoxy-4-(4-met hyl-1H-imidazol-1-yl)benzylidene]piperidin-2-one,
(E)-(4R,9aS)-7-[3-methoxy-4-(4-methyl-1H-imidazol-1-yl)benzylidene] -4-(3,4,5-trifluorophenyl)hexahydropyrido[2,1-c][1,4]oxazin-6-one, 3-[3-methoxy-4-(4-methyl-1H-imidazol-1-yl)benzylidene]-1-[(1S)-1-ph enylethyl]piperidin-2-one,
(E)-N-cyclohexylmethyl-3-[4-(1H-imidazol-1-yl)-3-methoxyphenyl]acr ylamide,
(E)-3-[4-(1H-imidazol-1-yl)-3-methoxyphenyl]-N-(1-phenylcyclopropyl )acrylamide,
(E)-3-[4-(1H-imidazol-1-yl)-3-trifluorophenyl]-N-indan-1-yl-acrylamid e,
(E)-3-[3-acetyl-4-(1H-imidazol-1-yl)-phenyl]-N-indan-1-yl-acrylamide,
(E)-3-[3-fluoro-4-(1H-imidazol-1-yl)-phenyl]-2-butenoic acid indan-1-yl-amide,
(E)-3-[4-(1H-imidazol-1-yl)-3-methoxyphenyl]-N-(1,2,3,4-tetrahydrona phthalen-1-yl)acrylamide,
(E)-3-[4-(1H-imidazol-1-yl)-3-methoxymethylphenyl]-N-mdan-1-yl-acr ylamide,
and their pharmaceutically acceptable salts.

6. The therapeutic agent for HCV infectious disease according to any one of claims 2 to 5, wherein the other therapeutic agent for HCV infectious disease is Interferon.

7. The therapeutic agent for HCV infectious disease according to claim 6, wherein the Interferon is Interferon-α-2b.

8. The therapeutic agent for HCV infectious disease according to any one of claims 2 to 5, wherein the other therapeutic agent for HCV infectious disease is Ribavirin.

9. The therapeutic agent for HCV infectious disease according to any one of claims 2 to 8, wherein the other therapeutic agent for HCV infectious disease is both Interferon and Ribavirin.

10. The therapeutic agent for HCV infectious disease according to any one of claims 2 to 5, wherein the other therapeutic agent for HCV infectious disease is an HCV protease inhibitor or HCV polymerase inhibitor.

11. A kit for therapy of HCV infectious disease, which comprises the compound represented by formula (I) or the pharmaceutically acceptable salt thereof according to claim 1, and another therapeutic agent for HCV infectious disease.

12. A use of the compound represented by formula (I) or the pharmaceutically acceptable salt thereof according to claim 1, for production of a therapeutic agent for HCV infectious disease comprising the compound or the pharmaceutically acceptable salt thereof.

13. The use according to claim 12, wherein the therapeutic agent for HCV infectious disease comprises the compound or the pharmaceutically acceptable salt thereof, and another therapeutic agent for HCV infectious disease.

14. A use of the compound represented by formula (I) or the pharmaceutically acceptable salt thereof according to claim 1 for production of a therapeutic agent for HCV infectious disease comprising the compound or the pharmaceutically acceptable salt thereof, and another therapeutic agent for HCV infectious disease being intended for combined use with.

15. The use according to claim 14, wherein the therapeutic agent for HCV infectious disease which is for administration simultaneously or successively with another therapeutic agent for HCV infectious disease.

16. A therapeutic method for HCV infectious disease comprising administration of the compound represented by formula (I) or the pharmaceutically acceptable salt thereof according to claim 1 in need of the subject.

17. The therapeutic method according to claim 16, wherein the compound or the pharmaceutically acceptable salt thereof is to be used in combination with another therapeutic agent for HCV infectious disease.

18. The therapeutic method according to claim 17, wherein the compound or the pharmaceutically acceptable salt thereof and another therapeutic agent for HCV infectious disease are administered simultaneously or successively.
